# EUROPEAN PATENT SPECIFICATION

(11) **EP 2 531 169 B1**
(45) Date of publication and mention of the grant of the patent: **03.12.2014**
(21) Application number: 11700359.0
(22) Date of filing: 18.01.2011
(51) Int. Cl.: A61K 8/60, A61Q 5/02, A61Q 5/10, A61K 8/04, A61K 8/42, A61K 8/44, A61Q 5/12, A61K 8/34, A61K 8/41, A61K 8/46, A61Q 5/00

(54) **Shampoo containing a gel network**
Shampoo enthaltend ein Gelnetzwerk
Composition de shampooing contenant un réseau de gel

(30) Priority: 01.02.2010 EP 10152233
(43) Date of publication of application: 12.12.2012
(73) Proprietor: Unilever PLC, A Company Registered in England and Wales under Company no. 41424, London EC4Y 0DY Greater London (GB); Unilever N.V., 3013 AL Rotterdam (NL)
(72) Inventor: MURRAY, Andrew, Malcolm, Wirral Merseyside CH63 3JW (GB); PHAM, Thuy-Anh, Wirral Merseyside CH63 3JW (GB)
(74) Representative: James, Helen Sarah
(86) International application number: PCT/EP2011/050614
(87) International publication number: WO 2011/092083

(56) References cited:
- WO-A1-2005/034895
- US-A- 3 964 500
- US-A1- 2003 228 334
- US-A1- 2006 024 256
- US-A1- 2008 187 507

## Description

The present invention relates to a composition comprising an esterified sucrose.

Compositions comprising esterified sugars are disclosed in US 932610 and US3950510.

Composition containing a conditioning gel network are disclosed in co-pending application EP08168600.

There however remains a need to improve the performance of shampoos containing esterified sugars with regard to product stability, especially at raised temperatures.

Accordingly, the present invention provides a shampoo composition comprising:
i) a cleaning phase comprising a cleansing anionic surfactant which is a salt and comprises an alkyl group with from 8 to 14 carbons;
ii) an aqueous conditioning gel network having no overall charge or is anionic, the gel network comprising:
   (a) fatty material;
   (b) a gel network anionic surfactant comprising an alkyl group with from 16 to 30 carbons;
   (c) cationic surfactant; and
iii) an esterified sucrose, having an alkyl carbon chain from C2 to C6.

The invention further relates to a method of manufacturing a shampoo composition comprising the steps of
i) forming a an aqueous conditioning gel network having no overall charge or is anionic, the gel network comprising:
   (a) fatty material;
   (b) a gel network anionic surfactant comprising an alkyl group with from 16 to 30 carbons;
   (c) cationic surfactant;
ii) adding the resulting gel network to diluted primary surfactant solution; and
iii) adding an esterified sucrose having an alkyl carbon chain from C2 to C6 to the resulting composition.

Further disclosed is a method of manufacturing a shampoo composition comprising the steps of
i) forming a an aqueous conditioning gel network having no overall charge or is anionic, the gel network comprising:
   (a) fatty material;
   (b) a gel network anionic surfactant comprising an alkyl group with from 16 to 30 carbons;
   (c) cationic surfactant;
   (d) an esterified sucrose having an alkyl carbon chain from C2 to C6; and
ii) adding the resulting gel network to diluted primary surfactant solution.

### The Gel Network

Preferably, the number of carbons of the anionic and cationic surfactants in the gel network are within 4, preferably 2 carbons of each other and most preferably have the same number of carbons. More preferably, the surfactants comprise a single alkyl group of within 4 carbons, more preferably within 2 carbons and most preferably are the same length. This assists in maintaining stability of the gel network.

Preferably, the carbons in the gel network cationic surfactant are present in a single alkyl group. More preferably the gel network cationic surfactant has from 16-30 carbons.

Preferably, the cationic surfactants have the formula N⁺(R¹)(R²)(R³)(R⁴), wherein R¹, R², R³ and R⁴ are independently (C₁₆ to C₃₀) alkyl or benzyl.

Preferably, one, two or three of R¹, R², R³ and R⁴ are independently (C₁₆ to C₃₀) alkyl and the other R¹, R², R³ and R⁴ group or groups are (C₁-C₆) alkyl or benzyl.

Optionally, the alkyl groups may comprise one or more ester (-OCO- or-COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (e.g., oleyl). Alkyl groups are optionally ethoxylated on the alkyl chain with one or more ethyleneoxy groups.

Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, stearyldimethylbenzylammonium chloride, cocotrimethylammonium chloride, PEG-2-oleammonium chloride and the corresponding hydroxides thereof. Further suitable cationic surfactants include those materials having the CTFA designations Quatemium-5, Quatemium-31 and Quatemium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant.

Another example of a class of suitable cationic surfactants for use in the invention, either alone or in admixture with one or more other cationic conditioning surfactants, is a combination of (i) and (ii) below:
(i) an amidoamine corresponding to the general formula (I): 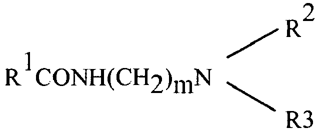
   in which R¹ is a hydrocarbyl chain having 10 or more carbon atoms,
   R² and R³ are independently selected from hydrocarbyl chains of from 1 to 10 carbon atoms, and
   m is an integer from 1 to about 10; and
(ii) an acid.

As used herein, the term hydrocarbyl chain means an alkyl or alkenyl chain.

Preferred amidoamine compounds are those corresponding to formula (I) in which

R¹ is a hydrocarbyl residue having from about 11 to about 24 carbon atoms, R² and R³ are each independently hydrocarbyl residues, preferably alkyl groups, having from 1 to about 4 carbon atoms, and m is an integer from 1 to about 4.

Preferably, R² and R³ are methyl or ethyl groups.

Preferably, m is 2 or 3, i.e. an ethylene or propylene group.

Preferred amidoamines useful herein include stearamido-propyldimethylamine, stearamidopropyldiethylamine, stearamidoethyldiethylamine, stearamidoethyldimethylamine, palmitamidopropyldimethylamine, palmitamidopropyldiethylamine, patmitamidoethyldiethylamine, palmitamidoethyldimethylamine, behenamidopropyldimethylamine, behenamidopropyldiethylmine, behenamidoethyldiethylamine, behenamidoethyldimethylamine, arachidamidopropyldimethylamine, arachidamidopropyldiethylamine, arachid-amidoethyldiethylamine, arachidamidoethyldimethylamine, and mixtures thereof.

Particularly preferred amidoamines useful herein are stearamidopropyldimethylamine, stearamidoethyldiethylamine, and mixtures thereof.

Commercially available amidoamines useful herein include:
stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a tradename INCROMINE BB available from Croda (North Humberside, England),
and various amidoamines with tradenames SCHERCODINE series available from Scher (Clifton New Jersey, USA).

Acid (ii) may be any organic or mineral acid which is capable of protonating the amidoamine in the hair treatment composition. Suitable acids useful herein include hydrochloric acid, acetic acid, tartaric acid, fumaric acid, lactic acid, malic acid, succinic acid, and mixtures thereof. Preferably, the acid is selected from the group consisting of acetic acid, tartaric acid, hydrochloric acid, fumaric acid, and mixtures thereof.

The primary role of the acid is to protonate the amidoamine in the hair treatment composition thus forming a tertiary amine salt (TAS) *in situ* in the hair treatment composition. The TAS in effect is a non-permanent quaternary ammonium or pseudo-quaternary ammonium cationic surfactant.

Suitably, the acid is included in a sufficient amount to protonate all the amidoamine present, i.e. at a level which is at least equimolar to the amount of amidoamine present in the composition.

The level of cationic surfactant will generally range from 0.01 to 10%, more preferably 0.02 to 7.5%, most preferably 0.05 to 5% by total weight of cationic surfactant based on the total weight of the composition.

The anionic surfactant comprises an alkyl chain with from 16-30 carbons, preferably from 16-22 carbons.

The anionic surfactant is preferably not a long chain fatty acid soap.

Preferably, the carbons in the gel network anionic surfactant are present in a single alkyl group.

Preferably the anionic salt is a sulphate, sulphonate, sarcosinate or isethionate, more preferably a sulphate, most preferably a sodium cetylstearyl sulphate.

The gel network comprises an anionic surfactant for achieving an overall anionic charge to the gel network or no overall charge to the gel network.

The gel network anionic surfactant is present at from 0.1 to 5 % by weight of the composition and more preferably from 0.5 to 2.0% wt.

The gel network comprises a fatty material.

Preferably, the fatty material is selected from fatty amides, fatty alcohols, fatty esters and mixtures thereof. Preferably, the fatty material is a fatty alcohol.

Preferably, the fatty material comprises a fatty group having from 14 to 30 carbon atoms, more preferably 16 to 22. Examples of suitable fatty alcohols include cetyl alcohol, stearyl alcohol and mixtures thereof. An example of a suitable fatty ester is glyceryl monostearate.

The level of fatty material in compositions of the invention is conveniently from 0.01 to 10%, preferably from 0.1 to 5% by weight of the composition.

Preferably the ratio between (a) and (b) is from 0.1:1 to 100:1, preferably from 1.2:1 to 50:1, more preferably from 1.5:1 to 10:1 and most preferably around 2:1.

Preferably, the anionic and fatty materials of the gel network contain alkyl groups with within 4, preferably 2 carbons and most preferably the same number of carbons. More preferably, they comprise a single alkyl group of within 4, more preferably within 2 and most preferably are the same length. This assists in maintaining stability of the gel network.

### The Esterified Sugar

Compositions of the invention comprise an esterified sugar having an alkyl carbon chain from C2 to C6.

It is further preferred if the esterified sugar is a sucrose, particularly preferred is sucrose acetate isobutyrate (SAIB).

Preferably the non-surfactant esterified sugar is non-crystalline at 20°C.

The level of esterified sugar in the composition is preferably from 0.001 to 10 wt% of the total composition, more preferably from 0.01 to 5 wt%, most preferably from 0.02 to 2 wt%.

It is further preferred if the level of esterified sugar deposited on the hair is from 0.3 to 12 mg of per g of hair, preferably from 0.6 to 6 mg, more preferably form 1 to 1.5 mg.

It is preferred if the non-surfactant esterified sugar is delivered from compositions comprising a solvent for the sugar. Suitable solvents include ethanol, isopropyl alcohol, n-propyl alcohol, n-butyl alcohol isobutyl alcohol, caprylic / capric triglycerides or mixtures thereof. Water may also be present with these solvents. Particularly preferred is ethanol, of particular benefit is an ethanol and water mix.

It is preferred if the weight ratio of sucrose acetate isobutyrate to solvent is from 0.01:100 to 1:100, more preferably from 2:1 to 1:40, most preferably from 1:1 to 1:20.

### Cleansing Phase

The cleaning phase comprises a cleansing surfactant. The cleansing phase anionic surfactant is a salt and has from 8 to 14 carbons, more preferably from 10 to 12 and most preferably 12 carbons. More preferably, these carbons are present in a single alkyl group.

Preferably, the salt is a sulphate, sulphonate, sarcosinate or isethionate.

Preferably, the cleansing anionic surfactant is selected from ammonium lauryl sulphate, ammonium laureth sulphate, trimethylamine lauryl sulphate, trimethylamine laureth sulphate, triethanolamine lauryl sulphate, trimethylethanolamine laureth sulphate, monoethanolamine lauryl sulphate, monoethanolamine laureth sulphate, diethanolamine lauryl sulphate, diethanolamine laureth sulphate, lauric monoglyceride sodium sulphate, sodium lauryl sulphate, sodium laureth sulphate, potassium lauryl sulphate, potassium laureth sulphate, sodium lauryl sarcosinate, sodium lauroyl sarcosinate, lauryl sarcosine, ammonium cocoyl sulphate, ammonium lauroyl sulphate, sodium cocoyl sulphate, sodium lauryl sulphate, potassium cocoyl sulphate, potassium lauryl sulphate, monoethanolamine cocoyl sulphate, monoethanolamine lauryl sulphate, sodium tridecyl benzene sulphonate, sodium dodecyl benzene sulphonate, sodium cocoyl isethionate and mixtures thereof.

Preferred anionic cleansing surfactants include alkali metal alkyl sulphates, more preferably the alkyl ether sulphates. Particularly preferred anionic cleansing surfactants include sodium lauryl ether sulphate.

The cleansing phase comprises from 0.5 to 70% by weight cleansing surfactant, preferably from 5 to 60% and more preferably from 7 to 56% by weight of the composition.

The preferred cleansing anionic surfactants provide a surfactant benefit no matter the pH of the composition since they are pH insensitive.

The invention encompasses both regular shampoo compositions comprising typical levels of cleansing surfactant as well as concentrated shampoos. In a regular shampoo the level of cleansing surfactant is from 5 to 26% by weight of the composition while for concentrated shampoos the level of cleansing surfactant is from 27 to 70% by weight.

Preferably, the composition comprises no fatty acid. Preferably the composition comprises no fatty acid having from 10 to 20 carbon atoms in an alkyl chain. Fatty acids are not desirable since they provide a poor quality conditioning benefit to the hair.

### Deposition Polymer

In a preferred embodiment the composition according to the invention comprises a cationic deposition polymer.

Suitable cationic deposition aid polymers may be homopolymers which are cationically substituted or may be formed from two or more types of monomers. The weight average (M_{w}) molecular weight of the polymers will generally be between 100 000 and 2 million daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerised in the amine form and then converted to ammonium by quaternization.

The cationic polymers can comprise mixtures of monomer units derived from amine- and/or quaternary ammonium-substituted monomer and/or compatible spacer monomers.

Suitable cationic polymers include, for example:
- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides(as described in WO95/22311).

Other cationic polymers that can be used include cationic polysaccharide polymers, such as cationic cellulose derivatives, cationic starch derivatives, and cationic guar gum derivatives.

Cationic polysaccharide polymers suitable for use in compositions of the invention include monomers of the formula:

A-O-[R-N⁺(R¹)(R²)(R³)X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14, JAGUAR C15 and JAGUAR C17.

Mixtures of any of the above cationic polymers may be used.

Cationic polymer will generally be present in a shampoo composition of the invention at levels of from 0.01 to 5%, preferably from 0.05 to 2%, more preferably from 0.07 to 1.2% by total weight of cationic polymer based on the total weight of the composition.

### Solvent

Preferably, the hair care compositions of the invention are aqueous, i.e. they have water or an aqueous solution or a lyotropic liquid crystalline phase as their major component.

Suitably, the composition will comprise from 10 to 98%, preferably from 30 to 95% water by weight based on the total weight of the composition.

### Silicone

The composition according to the invention preferably comprises a silicone.

Particularly preferred silicone conditioning agents are silicone emulsions such as those formed from silicones such as polydiorganosiloxanes, in particular polydimethylsiloxanes which have the CTFA designation dimethicone, polydimethyl siloxanes having hydroxyl end groups which have the CTFA designation dimethiconol, and amino-functional polydimethyl siloxanes which have the CTFA designation amodimethicone.

The emulsion droplets may typically have a Sauter mean droplet diameter (D_{3,2}) in the composition of the invention ranging from 0.01 to 20 micrometer, more preferably from 0.2 to 10 micrometer.

A suitable method for measuring the Sauter mean droplet diameter (D_{3,2}) is by laser light scattering using an instrument such as a Malvern Mastersizer.

Suitable silicone emulsions for use in compositions of the invention are available from suppliers of silicones such as Dow Corning and GE Silicones. The use of such pre-formed silicone emulsions is preferred for ease of processing and control of silicone particle size. Such pre-formed silicone emulsions will typically additionally comprise a suitable emulsifier such as an anionic or nonionic emulsifier, or mixture thereof, and may be prepared by a chemical emulsification process such as emulsion polymerisation, or by mechanical emulsification using a high shear mixer. Pre-formed silicone emulsions having a Sauter mean droplet diameter (D_{3,2}) of less than 0.15 micrometers are generally termed microemulsions.

Examples of suitable pre-formed silicone emulsions include emulsions DC2-1766, DC2-1784, DC-1785, DC-1786, DC-1788 and microemulsions DC2-1865 and DC2-1870, all available from Dow Corning. DC7051 is a preferred silicone. These are all emulsions/microemulsions of dimethiconol. Also suitable are amodimethicone emulsions such as DC2-8177 and DC939 (from Dow Corning) and SME253 (from GE Silicones).

Also suitable are silicone emulsions in which certain types of surface active block copolymers of a high molecular weight have been blended with the silicone emulsion droplets, as described for example in WO03/094874. In such materials, the silicone emulsion droplets are preferably formed from polydiorganosiloxanes such as those described above. One preferred form of the surface active block copolymer is according to the following formula:

HO(CH₂CH₂O)ₓ(CH(CH₃)CH₂O)_{y}(CH₂CH₂O)ₓ H

wherein the mean value of x is 4 or more and the mean value of y is 25 or more.

Another preferred form of the surface active block copolymer is according to the following formula:

(HO(CH₂CH₂O)ₐ(CH(CH₃)CH₂O)_{b})₂-N-CH₂-CH₂-N((OCH₂CH(CH₃))_{b}(OCH₂CH₂)ₐ OH)₂

wherein the mean value of a is 2 or more and the mean value of b is 6 or more.

Mixtures of any of the above described silicone emulsions may also be used.

The above described silicone emulsions will generally be present in a composition of the invention at levels of from 0.05 to 15%, preferably from 0.5 to 12% by total weight of silicone based on the total weight of the composition.

The silicone is preferably present at from 0.5 to 15% wt., more preferably 1 to 12% by weight.

Optionally, a composition of the invention may contain further ingredients as described below to enhance performance and/or consumer acceptability.

The composition can include co-surfactants, to help impart aesthetic, physical or cleansing properties to the composition.

An example of a co-surfactant is a nonionic surfactant, which can be included in an amount ranging from 0.5 to 10%, preferably from 0.7 to 6% by weight based on the total weight of the composition.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Other representative nonionic surfactants include mono- or di-alkyl alkanolamides. Examples include coco mono- or di-ethanolamide and coco monoisopropanolamide. A particularly preferred nonionic surfactant is coco monoethanolamide.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO - (G)n

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies from about 1.1 to about 2. Most preferably the value of n lies from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the C₁₀-C₁₈ N-alkyl (C_{I}-C₆) polyhydroxy fatty acid amides, such as the C₁₂-C₁₈N-methyl glucamides, as described for example in WO 92 06154 and US 5 194 639, and the N-alkoxy polyhydroxy fatty acid amides, such as C₁₀-C₁₈ N-(3-methoxypropyl) glucamide.

A preferred example of a co-surfactant is an amphoteric or zwitterionic surfactant, which can be included in an amount ranging from 0.5 to about 10%, preferably from 1 to 6% by weight based on the total weight of the composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocamidopropyl betaine.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

The total amount of surfactant (including any co-surfactant, and/or any emulsifier) in a shampoo composition of the invention is generally from 1 to 70%, preferably from 2 to 65%, more preferably from 8 to 60% by total weight surfactant based on the total weight of the composition.

### Suspending Agent

Preferably an aqueous shampoo composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

Suspending agent will generally be present in a shampoo composition of the invention at levels of from 0.1 to 10%, preferably from 0.5 to 6%, more preferably from 0.9 to 4% by total weight of suspending agent based on the total weight of the composition.

Preferably, the composition has a viscosity of 2000 to 7000 cPs measures at 30°C, measured on a Brookfield Viscometer using spindle RV5 at 20 rpm.

### Oil

The composition preferably comprises an oil. The oil may be any oil commonly used in personal care products for example polyolefin oils, ester oils, triglyceride oils, hydrocarbon oils and mixtures thereof. Preferably, the oil is a light oil. Oils, enhance the conditioning benefits found with compositions of the invention.

Preferred oils include those selected from:
- Oils having viscosities from 0.1 to 500 centipoises measures at 30°C.
- Oils with viscosity above 500 centipoises (500-500000 cps) which contains up to 20% of a lower viscosity fraction (less than 500cps).

One type of preferred oil is a polyalphaolefin oil.

Suitable polyalphaolefin oils include those derived from 1-alkalene monomers having from 6 to 16 carbons, preferably from 6 to 12 carbons. Non limiting examples of materials include 1-hexene, 1-octene, 1-decene, 1-dodecene, 1-tetradecene, 1-hexadecene, branched isomers such as 4-methyl-1-pentene and mixtures thereof.

Preferred polyalphaloefins include polydecenes with tradename Puresyn 6 having a number average molecular weight of about 500, Puresyn 100 having a molecular weight of about 3000 and Puresyn 300 having a molecular weight of about 6000 commercially available from Mobil.

Preferably, the polyalphaolefin oil is present at from 0.05 to 10%, particularly from 0.2 to 5%, and especially from 0.5 to 3% by weight of the composition.

Also suitable are triglyceride oils include fats and oils including natural fats and oils such as jojoba, soybean, sunflower seed oil, rice bran, avocado, almond, olive, sesame, castor, coconut, coconut palm oil, sunflower oil, mink oils; cacao fat; beef tallow, lard; hardened oils obtained by hydrogenating the aforementioned oils; and synthetic mono, di- and triglycerides such as myristic acid glyceride and 2-ethylhexanoic acid glyceride.

Preferably, the triglyceride oil if present is at levels from 0.05 to 10%, particularly from 0.2 to 5%, and especially from 0.5 to 3% by weight of the composition. Highly suitable oils for use with the present invention are hydrocarbon oils. Hydrocarbon oils have at least 12 carbon atoms, and include paraffin oil, polyolefin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used. Also suitable are polymeric hydrocarbons of C₂₋₆ alkenyl monomers, such as polyisobutylene.

Preferably, the hydrocarbon oil is present at from 0.05 to 10%, particularly from 0.2 to 5%, and especially from 0.5 to 3% by weight of the composition.

Also suitable are ester oils with have at least 10 carbon atoms, and include esters with hydrocarbyl chains derived from fatty acids or alcohols. Typical ester oils are formula R'COOR in which R' and R independently denote alkyl or alkenyl radicals and the sum of carbon atoms in R' and R is at least 10, preferably at least 20. Diand trialkyl and alkenyl esters of carboxylic acids can also be used.

Preferably, the ester oil is present at from 0.05 to 10%, particularly from 0.2 to 5%, and especially from 0.5 to 3% by weight of the composition.

Preferably, the composition comprises a cleansing anionic surfactant which comprises an alkyl group with from 10 to 14 carbons.

A further component that may be used in compositions of the invention is a hydrocarbon oil or ester oil. Like silicone oils, these materials may enhance the conditioning benefits found with compositions of the invention.

Suitable hydrocarbon oils have at least 12 carbon atoms, and include paraffin oil, polyolefin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used. Also suitable are polymeric hydrocarbons of C₂₋₆ alkenyl monomers, such as polyisobutylene.

Suitable ester oils have at least 10 carbon atoms, and include esters with hydrocarbyl chains derived from fatty acids or alcohols. Typical ester oils are formula R'COOR in which R' and R independently denote alkyl or alkenyl radicals and the sum of carbon atoms in R' and R is at least 10, preferably at least 20. Diand trialkyl and alkenyl esters of carboxylic acids can also be used.

Mixtures of any of the above described hydrocarbon/ester oils also be used.

The total combined amount of hydrocarbon oil and ester oil in compositions of the invention may suitably range from 0.05 to 10%, particularly from 0.2 to 5%, and especially from 0.5 to 3% by weight of the composition.

### Other Ingredients

A composition of the invention may contain other ingredients for enhancing performance and/or consumer acceptability. Such ingredients include fragrance, dyes and pigments, pH adjusting agents, pearlescers or opacifiers, viscosity modifiers, and preservatives or antimicrobials. Each of these ingredients will be present in an amount effective to accomplish its purpose. Generally these optional ingredients are included individually at a level of up to 5% by weight of the total composition.

The invention will be further illustrated by the following, non-limiting Example, in which all percentages quoted are by weight based on total weight unless otherwise stated.

### Examples

| **Component** | **%ad** | **Comparative** | **Example 1** | **Example 2** | **Example 3** | **Example 4** | **Example 5** |
|---|---|---|---|---|---|---|---|
| Sodium Laureth Sulphate | 70 | 17.14 | 17.14 | 17.14 | 17.14 | 17.14 | 17.14 |
| Cocoamidopropyl Betaine | 30 | 5.33 | 5.33 | 5.33 | - | - | 5.33 |
| Cocamide MEA | 85 | - | - | - | 1.0 | 1.0 | - |
| Carbomer | 100 | 0.4 | 0.4 | 0.4 | 0.4 | 0.4 | 0.4 |
| Glycol Distearate | 35 | 4.0 | 4.0 | 4.0 | 4.0 | 4.0 | 4.0 |
| Dimethiconol | 50 | 4.0 | 4.0 | 4.0 | 4.0 | 4.0 | 4.0 |
| Sodium Cetylstearyl sulphate | 100 | - | 0.6 | 0.6 | 0.6 | 0.6 | 0.6 |
| Cetostearyl Alcohol | 100 | - | 1.0 | 1.0 | 1.0 | 1.0 | 1.0 |
| Cetyl trimethylammonium chloride | 29 | - | - | 0.17 | - | 0.17 | - |
| Behenyl Trimethyl Ammonium Chloride | 77.5 | - | 0.06 | - | 0.06 | - | 0.06 |
| Sucrose acetate isobutyrate in ethanol (10%) | 90 | 0.333 | 0.333 | 0.333 | 0.333 | 0.333 | 0.333 |
| Mineral oil | 100 | - | - | - | - | - | 0.3 |
| Guar Hydroxypropyl Trimonium Chloride | 100 | 0.2 | 0.2 | 0.2 | 0.2 | 0.2 | 0.2 |
| Parfume | 100 % | 0.8 | 0.8 | 0.8 | 0.8 | 0.8 | 0.8 |
| DMDM Hydantoin and 3-iodo-2propylnylbutyl carbamate | 50% | 0.2 | 0.2 | 0.2 | 0.2 | 0.2 | 0.2 |
| Sodium chloride | 100 % | Visc. | Visc. | Visc. | Visc. | Visc. | Visc. |
| Aqua | | q.s.to 100 | q.s.to 100 | q.s.to 100 | q.s.to 100 | q.s.to 100 | q.s.to 100 |

| **Component** | **%ad** | **Comparative** | **Example 6** | **Example 7** | **Example 8** | **Example 9** | **Example 10** |
|---|---|---|---|---|---|---|---|
| Sodium Laureth Sulphate | 70 | 17.14 | 17.14 | 17.14 | 17.14 | 17.14 | 17.14 |
| Cocoamidopropyl Betaine | 30 | 5.33 | 5.33 | 5.33 | - | - | 5.33 |
| Cocamide MEA | 85 | - | - | - | 1.0 | 1.0 | - |
| Carbomer | 100 | 0.4 | 0.4 | 0.4 | 0.4 | 0.4 | 0.4 |
| Glycol Distearate | 35 | 4.0 | 4.0 | 4.0 | 4.0 | 4.0 | 4.0 |
| Dimethiconol | 50 | 4.0 | 4.0 | 4.0 | 4.0 | 4.0 | 4.0 |
| Sodium Cetylstearyl sulphate | 100 | - | 0.6 | 0.6 | 0.6 | 0.6 | 0.6 |
| Cetostearyl Alcohol | 100 | - | 1.0 | 1.0 | 1.0 | 1.0 | 1.0 |
| Cetyl trimethylammonium chloride | 29 | - | - | 0.17 | - | 0.17 | - |
| Behenyl Trimethyl Ammonium Chloride | 77.5 | - | 0.06 | - | 0.06 | - | 0.06 |
| Sucrose acetate isobutyrate in caprilyc and capric triglycerides (20%) | 80 | 0.375 | 0.375 | 0.375 | 0.375 | 0.375 | 0.375 |
| Mineral oil | 100 | - | - | - | - | - | 0.3 |
| Guar Hydroxypropyl Trimonium Chloride | 100 | 0.2 | 0.2 | 0.2 | 0.2 | 0.2 | 0.2 |
| Parfum | 100% | 0.8 | 0.8 | 0.8 | 0.8 | 0.8 | 0.8 |
| DMDM Hydantoin and 3-iodo-2propylnylbutyl carbamate | 50% | 0.2 | 0.2 | 0.2 | 0.2 | 0.2 | 0.2 |
| Sodium chloride | 100% | Visc. | Visc. | Visc. | Visc. | Visc. | Visc. |
| Aqua | | q.s.to 100 | q.s.to 100 | q.s.to 100 | q.s.to 100 | q.s.to 100 | q.s.to 100 |

### Process 1

At least 7% of water was heated to about 80°C in a side pot. To this, was added cationic surfactant (Behenyl Trimethyl Ammonium Chloride), fatty alcohol, sucrose acetate isobutyrate, and secondary anionic surfactant (Sodium Cetylstearyl sulphate) using high speed stirring. When uniform dispersion obtained, this mixture was cooled down to about 45°C with the same speed stirring. This mixture was then added in the diluted primary surfactant solution (Sodium Laureth Sulphate) following by remaining components with moderate speed stirring.

### Process 2

At least 7% of water was heated to about 80°C in a side pot. To this, was added cationic surfactant (Behenyl Trimethyl Ammonium Chloride), fatty alcohol, and secondary anionic surfactant (Sodium Cetylstearyl sulphate) using high speed stirring. When uniform dispersion obtained, this mixture was cooled down to about 45°C with the same speed stirring. This mixture was then added in the diluted primary surfactant solution (Sodium Laureth Sulphate) following by remaining components with moderate speed stirring, add sucrose acetate isobutyrate after adding salt.

### Results

The comparative shampoo with 0.3%SAIB but no gel phase is not stable at 45°C storage after 12 weeks storage. The examples of the invention using a conditioning gel network are stable after 12 weeks storage at 45°C.

It should also be noted that formulations with sucrose acetate isobutyrate give good conditioning benefits to hair. The inclusion of oil further enhances these conditioning benefits.

## Claims

1. A shampoo composition comprising:
i) a cleaning phase comprising a cleansing anionic surfactant which is a salt and comprises an alkyl group with from 8 to 14 carbons;
ii) an aqueous conditioning gel network having no overall charge or is anionic, the gel network comprising:
(a) fatty material;
(b) a gel network anionic surfactant comprising an alkyl group with from 16 to 30 carbons;
(c) cationic surfactant; and
iii) an esterified sucrose, having an alkyl carbon chain from C2 to C6.

2. A composition according to claim 1 wherein the testified sucrose is sucrose acetate isobutyrate.

3. A composition according to any preceding claim in which the composition further comprises an oil.

4. A composition according to claim 3 in which the oil is mineral oil.

5. A composition according to any preceding claim wherein the fatty material is selected from fatty alcohols, fatty esters, fatty acids and fatty amides.

6. A composition according to any preceding claim wherein the fatty material is straight chain or branched and has from 14 to 30 carbons.

7. A composition according to any preceding claim wherein the gel network anionic surfactant has from 16 to 22 carbons.

8. A composition according to any preceding claim wherein the gel network cationic surfactant has from 16 to 30 carbons.

9. A composition according to any preceding claim wherein the ratio between (a) and (b) is from 0.1:1 to 100:1, preferably from 1.2:1 to 50:1, more preferably from 1.5:1 to 10:1 and most preferably around 2:1.

10. A composition according to any preceding claim comprising a cationic deposition polymer.

11. A composition according to any preceding claim in which the composition further comprises a suspending agent.

12. A composition according to any preceding claim comprising a silicone.

13. A composition according to any preceding claim wherein the cleansing anionic surfactant is a sulphate, sulphonate, sarcosinate or isethionate.

14. A method of manufacturing a shampoo composition comprising the steps of
i) forming a an aqueous conditioning gel network having no overall charge or is anionic, the gel network comprising:
(a) fatty material;
(b) a gel network anionic surfactant comprising an alkyl group with from 16 to 30 carbons;
(c) cationic surfactant;
ii) adding the resulting gel network to diluted primary surfactant solution; and
iii) adding an esterified sucrose having an alkyl chain length from C2 to C6 to the resulting composition.

15. A method of manufacturing a shampoo composition comprising the steps of
i) forming a an aqueous conditioning gel network having no overall charge or is anionic, the gel network comprising:
(a) fatty material;
(b) a gel network anionic surfactant comprising an alkyl group with from 16 to 30 carbons;
(c) cationic surfactant;
(d) an esterified sucrose; having an alkyl chain length from C2 to C6 and
ii) adding the resulting gel network to diluted primary surfactant solution.

## Patentansprüche

1. Shampoozusammensetzung,
die Folgendes aufweist:
i) eine reinigende Phase, die ein reinigendes anionisches Tensid aufweist, das ein Salz ist und eine Alkylgruppe mit 8 bis 14 Kohlenstoffatomen aufweist;
ii) ein wässriges konditionierendes Gelnetzwerk, das insgesamt keine Ladung aufweist oder anionisch ist, wobei das Gelnetzwerk Folgendes aufweist:
(a) ein Fettmaterial;
(b) ein anionisches Tensid in Form eines Gelnetzwerks, das eine Alkylgruppe mit 16 bis 30 Kohlenstoffatomen aufweist;
(c) ein kationisches Tensid; und
iii) eine veresterte Saccharose mit einer Alkylkohlenstoffkette mit C₂ bis C₆.

2. Zusammensetzung nach Anspruch 1,
wobei besagte Saccharose Saccharoseacetatisobutyrat ist.

3. Zusammensetzung nach einem der vorstehenden Ansprüche,
wobei die Zusammensetzung ferner ein Öl aufweist.

4. Zusammensetzung nach Anspruch 3,
wobei das Öl Mineralöl ist.

5. Zusammensetzung nach einem der vorstehenden Ansprüche,
wobei das Fettmaterial aus Fettalkoholen, Fettestern, Fettsäuren und Fettamiden ausgewählt ist.

6. Zusammensetzung nach einem der vorstehenden Ansprüche,
wobei das Fettmaterial geradkettig oder verzweigt ist und 14 bis 30 Kohlenstoffatome aufweist.

7. Zusammensetzung nach einem der vorstehenden Ansprüche,
wobei das anionische Tensid in Form eines Gelnetzwerks 16 bis 22 Kohlenstoffatome aufweist.

8. Zusammensetzung nach einem der vorstehenden Ansprüche,
wobei das kationische Tensid in Form eines Gelnetzwerks 16 bis 30 Kohlenstoffatome aufweist.

9. Zusammensetzung nach einem der vorstehenden Ansprüche,
wobei das Verhältnis zwischen (a) und (b) 0,1:1 bis 100:1, vorzugsweise 1,2: bis 50:1, stärker bevorzugt 1,5:1 bis 10:1 und besonders bevorzugt etwa 2:1 beträgt.

10. Zusammensetzung nach einem der vorstehenden Ansprüche,
die ein kationisches Ablagerungspolymer aufweist.

11. Zusammensetzung nach einem der vorstehenden Ansprüche,
wobei die Zusammensetzung ferner ein Suspensionsmittel aufweist.

12. Zusammensetzung nach einem der vorstehenden Ansprüche,
die ein Silicon aufweist.

13. Zusammensetzung nach einem der vorstehenden Ansprüche,
wobei das reinigende anionische Tensid ein Sulfat, Sulfonat, Sarcosinat oder Isethionat ist.

14. Verfahren zum Herstellen einer Shampoozusammensetzung,
das die folgenden Schritte aufweist:
i) Erzeugen eines wässrigen konditionierenden Gelnetzwerks, das insgesamt keine Ladung aufweist oder anionisch ist, wobei das Gelnetzwerk Folgendes aufweist:
(a) ein Fettmaterial;
(b) ein anionisches Tensid in Form eines Gelnetzwerks, das eine Alkylgruppe mit 16 bis 30 Kohlenstoffatomen aufweist;
(c) ein kationisches Tensid;
ii) Zugeben des entstandenen Gelnetzwerks zu einer verdünnten primären Tensidlösung; und
iii) Zugeben einer veresterten Saccharose mit einer Alkylkettenlänge von C₂ bis C₆ zu der entstandenen Zusammensetzung.

15. Verfahren zum Herstellen einer Shampoozusammensetzung,
das die folgenden Schritte aufweist:
i) Erzeugen eines wässrigen konditionierenden Gelnetzwerks, das insgesamt keine Ladung aufweist oder anionisch ist, wobei das Gelnetzwerk Folgendes aufweist:
(a) ein Fettmaterial;
(b) ein anionisches Tensid in Form eines Gelnetzwerks, das eine Alkylgruppe mit 16 bis 30 Kohlenstoffatomen aufweist;
(c) ein kationisches Tensid;
(d) eine veresterte Saccharose mit einer Alkylkettenlänge von C₂ bis C₆; und
ii) Zugeben des entstandenen Gelnetzwerks zur verdünnten primären Tensidlösung.

## Revendications

1. Composition de shampooing comprenant :
i) une phase nettoyante comprenant un tensioactif anionique nettoyant qui est un sel et comprend un groupe alkyle ayant de 8 à 14 carbones ;
ii) un réseau de gel conditionnant aqueux n'ayant pas de charge globale ou est anionique, le réseau de gel comprenant :
(a) une matière grasse ;
(b) un tensioactif anionique de réseau de gel comprenant un groupe alkyle ayant de 16 à 30 carbones ;
(c) un tensioactif cationique ; et
iii) un saccharose estérifié ayant une chaîne carbonée alkyle de C2 à C6.

2. Composition selon la revendication 1 où le saccharose estérifié est l'acétate isobutyrate de saccharose.

3. Composition selon l'une quelconque des revendications précédentes où la composition comprend en outre une huile.

4. Composition selon la revendication 3 où l'huile est une huile minérale.

5. Composition selon l'une quelconque des revendications précédentes où la matière grasse est choisie parmi les alcools gras, les esters gras, les acides gras et les amides gras.

6. Composition selon l'une quelconque des revendications précédentes où la matière grasse est linéaire ou ramifiée et a de 14 à 30 carbones.

7. Composition selon l'une quelconque des revendications précédentes où le tensioactif anionique de réseau de gel a de 16 à 22 carbones.

8. Composition selon l'une quelconque des revendications précédentes où le tensioactif cationique de réseau de gel a de 16 à 30 carbones.

9. Composition selon l'une quelconque des revendications précédentes où le rapport entre (a) et (b) est de 0,1:1 à 100:1, de préférence de 1,2:1 à 50:1, de manière encore préférable de 1,5:1 à 10:1 et de manière particulièrement préférable d'environ 2:1.

10. Composition selon l'une quelconque des revendications précédentes comprenant un polymère de dépôt cationique.

11. Composition selon l'une quelconque des revendications précédentes où la composition comprend en outre un agent de suspension.

12. Composition selon l'une quelconque des revendications précédentes comprenant un silicone.

13. Composition selon l'une quelconque des revendications précédentes où le tensioactif anionique nettoyant est un sulfate, sulfonate, sarcosinate ou iséthionate.

14. Procédé de fabrication d'une composition de shampooing comprenant les étapes de
i) formation d'un réseau de gel conditionnant aqueux n'ayant pas de charge globale ou est anionique, le réseau de gel comprenant :
(a) une matière grasse ;
(b) un tensioactif anionique de réseau de gel comprenant un groupe alkyle ayant de 16 à 30 carbones ;
(c) un tensioactif cationique ;
ii) addition du réseau de gel résultant à une solution de tensioactif primaire diluée ; et
iii) addition d'un saccharose estérifié ayant une longueur de chaîne alkyle de C2 à C6 à la composition résultante.

15. Procédé de fabrication d'une composition de shampooing comprenant les étapes de
i) formation d'un réseau de gel conditionnant aqueux n'ayant pas de charge globale ou est anionique, le réseau de gel comprenant :
(a) une matière grasse ;
(b) un tensioactif anionique de réseau de gel comprenant un groupe alkyle ayant de 16 à 30 carbones ;
(c) un tensioactif cationique ;
(d) un saccharose estérifié ayant une longueur de chaîne alkyle de C2 à C6 ; et
ii) addition du réseau de gel résultant à une solution de tensioactif primaire diluée.
